# EUROPEAN PATENT SPECIFICATION

(11) **EP 2 002 824 B1**
(45) Date of publication and mention of the grant of the patent: **26.01.2011**
(21) Application number: 08252049.5
(22) Date of filing: 13.06.2008
(51) Int. Cl.: A61K 9/06

(54) **Gel composition and use thereof**
Gelzusammensetzung und ihre Verwendung
Composition de gel et utilisation correspondante

(30) Priority: 15.06.2007 JP 2007158669
(43) Date of publication of application: 17.12.2008
(73) Proprietor: Nitto Denko Corporation, Ibaraki-shi Osaka 567-8680 (JP)
(72) Inventor: Ishikura, Jun, Ibaraki-shi Osaka 567-8680 (JP); Kasahara, Tsuyoshi, Ibaraki-shi Osaka 567-8680 (JP); Hamada, Atsushi, Ibaraki-shi Osaka 567-8680 (JP); Funahashi, Miki, Ibaraki-shi Osaka 567-8680 (JP)
(74) Representative: Duckworth, Timothy John

(56) References cited:
- EP-A- 1 462 121
- EP-A- 1 625 846

## Description

### TECHNICAL FIELD OF THE INVENTION

The present invention relates to a gel composition, molded article, a sheet material, a patch and a patch preparation for a medical material or a hygiene material.

### BACKGROUND OF THE INVENTION

Conventionally, sheet materials of various gel compositions of, for example, a hydrogel composition, an acrylic gel composition and the like have been used as a medical material or a hygiene material. These gel compositions show adhesion to the skin and followability to skin deformation since they have adhesiveness and flexibility.

However, of these adhesive compositions, a hydrogel composition containing water is weak to dryness, tends to lose water over time and become hard and has poor stability. In addition, such a hardened composition is insufficient from the aspects of adhesion to the skin and followability to skin deformation.

On the other hand, as a gel material free of water, JP-A-3-220120 (patent reference 1) discloses an acrylic gel composition retaining an organic liquid component by crosslinking an acrylic adhesive.

However, since molding of such gel compositions into a sheet material having a certain level of thickness is difficult, the ability of the compositions to maintain or absorb a chemical substance such as a drug and the like, an organic liquid component and the like therein is still limitative, and the compositions cannot easily retain them in a large amount. Moreover, it is somewhat difficult to mold such a gel composition into a molded article with a large thickness. When a molded article with a small thickness is adhered to the skin, a further improvement may be demanded in its ability to absorb external impacts and protect the skin.

As other gel materials, an adhesive layer obtained by crosslinking a synthetic rubber polymer can be used. As such adhesive layer, the following have been proposed.

JP-A-3-127727 (patent reference 2) discloses an adhesive mainly constituted from a non-acrylic compound and having a glass transition temperature of -71°C or below, and an adhesive layer containing a reinforcing filler and a pharmaceutical agent, i.e., a gel composition. However, this reference does not disclose that 90 parts by weight or above of an organic liquid component is contained relative to 100 parts by weight of a rubber component. Moreover, this reference discloses that too much amount of an organic liquid component (described as a Tg temperature lowing material in the reference) to be added affords an excessively soft gel composition. This suggests that too much amount of an organic liquid component in the gel composition leads to insufficient shape retaining property. In fact, the Examples of this reference describes that adhesives stuck out 3 mm or below from one edge when applied to the human body.

JP-A-10-151185 (patent reference 3) discloses a gel composition containing 30 - 100 parts by weight of a rubber component having a functional group, 0 - 70 parts by weight of other rubbers, and 20 - 80 parts by weight of an organic liquid component compatible with the rubber component, relative to 100 parts by weight of the rubber component, wherein the gel composition is crosslinked. However, this reference does not disclose that 90 parts by weight or above of an organic liquid component is contained relative to 100 parts by weight of a rubber component. However, this reference discloses that too much amount of an organic liquid component to be added affords an excessively soft gel composition. This suggests that too much amount of an organic liquid component in the gel composition leads to insufficient shape retaining property.

EP-A-1 625 846 discloses a composition comprising polyisobutylene and isopropyl myristate which is used in the examples of the present invention.

EP-A-1 462 121 discloses a composition comprising a styrene-isoprene-styrene block copolymer (SIS) and liquid paraffin as the organic liquid component.

Therefore, the development of a composition for a medical material or a hygiene material, which contains a large amount of an organic liquid component, has sufficient flexibility and is capable of retaining shape, is desired.
patent reference 1: JP-A-3-220120
patent reference 2: JP-A-3-127727
patent reference 3: JP-A-10-151185

### Disclosure of the Invention

### Problems to be Solved by the Invention

In view of the above-mentioned, the present invention aims at providing a composition for a medical material or a hygiene material, which contains a large amount of an organic liquid component, has sufficient flexibility and is capable of retaining shape.

### Means of Solving the Problems

The present inventors have conducted intensive studies in an attempt to solve the above-mentioned problems and found that a gel composition containing a particular liquid rubber component and an organic liquid component at a given ratio can solve the above-mentioned problems. The present inventors have unexpectedly found that such gel composition can be molded into a form having a certain thickness, and the gel composition having such thickness can provide a new effect that it can contain a large amount of a chemical substance such as a drug and the like, an organic liquid component and the like per unit area, which resulted in the completion of the present invention. That is, the present invention provides:
(1) a gel composition for a medical material or a hygiene material, which comprises a liquid rubber component having 3 or more functional groups capable of crosslinking reaction in a molecule and 90 parts by weight or above and 1250 parts by weight or below of an organic liquid component relative to 100 parts by weight of the rubber component, which gel composition is crosslinked;
(2) the composition of (1), wherein the organic liquid component is contained in a proportion of 150 parts by weight or above and 1,000 parts by weight or below relative to 100 parts by weight of the liquid rubber component;
(3) the composition of any of (1) to (2), wherein the composition is crosslinked by a crosslinking agent, and the ratio of the total number (A) of the functional group of the crosslinking agent in the composition to the total number (B) of the functional group of the liquid rubber in the composition ((A/B)x100) [%] is not less than 50[%];
(4) the composition of any of (1) to (3), wherein the liquid rubber component is a liquid isoprene rubber;
(5) a patch having a sheet material containing the composition of any of (1) to (4) on at least one surface of a support; and
(6) a patch preparation comprising the patch of (5) containing a drug in the sheet material.

### Effect of the Invention

Since the gel composition of the present invention has sufficient flexibility and can retain shape, it can be molded into any form, for example, a sheet preparation.

In addition, since the gel composition of the present invention can contain a large amount of an organic liquid component, a large amount of a chemical substance such as a drug and the like can be contained by dissolution or dispersion therein. As a result, the chemical substance such as a drug and the like thus contained can be efficiently applied to a mammal by transdermal administration and the like.

Moreover, since the gel composition of the present invention has suitable flexibility, when it is molded into a given shape and applied to the skin as a molded article, it can easily follow skin deformation and can be adhered closely to the skin. In addition, since the composition can be molded into a molded article having a certain level of thickness, when the molded article is adhered to the skin, the stress applied to the skin can be dispersed or reduced to protect the skin. Thus, the gel composition of the present invention having such property is particularly suitable for a medical material or a hygiene material.

As mentioned above, since the gel composition of the present invention can be molded into a molded article having a certain level of thickness, the gel composition of the present invention having such thickness can provide a new effect that it can contain a large amount of a chemical substance, an organic liquid component and the like per unit area.

Furthermore, since the gel composition of the present invention does not require use of an acrylic adhesive, when a certain kind of a chemical substance such as a drug and the like is contained in the gel composition, the possibility of alteration of the substance due to an interaction between the substance and the acrylic adhesive is small, the degree of freedom of choice of the substance is high, and such substance can be used stably.

### Best Mode for Carrying out the Invention

The gel composition for a medical material or a hygiene material of the present invention contains a liquid rubber component having 3 or more functional groups capable of crosslinking reaction in a molecule, and an organic liquid component. The organic liquid component is contained in a proportion of 90 parts by weight or above and 1,250 parts by weight or below relative to 100 parts by weight of the rubber component. The gel composition is crosslinked.

In the present invention, the liquid rubber component plays the roles of forming a network structure upon crosslinking of the gel composition and imparting flexibility and shape retaining property to the gel composition.

The liquid rubber component is not particularly limited, and examples thereof include liquid isoprene rubbers, liquid butadiene rubbers, liquid isobutylene rubbers and the like, which may be used alone or in a combination of two or more kinds thereof. The liquid rubber component is liquid even when it has a high molecular weight, due to which the gel composition can easily contain a large amount of an organic liquid component and flexibility can be easily imparted to the gel composition. Therefore, a liquid isoprene rubber is preferable. The "liquid" in this specification means the presence of flowability at 25°C.

While the weight average molecular weight of the liquid rubber component is not particularly limited as long as the rubber component is a liquid, it is preferably 1,000 - 60,000, more preferably 10,000 - 40,000, most preferably 20,000 - 30,000. When it is less than 1,000, the gel composition cannot be easily contained in a large amount in the organic liquid component, which may decrease the flexibility of the gel composition. While the mechanism thereof is unknown, it is assumed that the network in the network structure of the liquid rubber component becomes small since the molecular weight between crosslinking points of the liquid rubber component decreases.

On the other hand, when it exceeds 60,000, the liquid rubber component may not be liquid, and further, the content uniformity of the gel composition may not be maintained easily. In other words, the compatibility of the liquid rubber component relative to other components of the gel composition may be difficult to secure.

The weight average molecular weight used herein means the value measured by gel permeation chromatography under the following conditions:
(analysis conditions)
GPC apparatus: HLC8120 (manufactured by Tosoh Corporation) column: TSK gel GMH-H(S) (manufactured by Tosoh Corporation) standard: polystyrene
eluent: tetrahydrofuran
flow rate: 0.5 ml/min
measurement temperature: 40°C
detection means: differential refractometer

Whether or not the gel composition of the present invention is crosslinked is determined as follows. A sample of a gel composition is immersed in toluene at ambient temperature for 6 days, and the presence of an insoluble fraction is visually confirmed. When an insoluble fraction is present, the gel composition is crosslinked.

Since the liquid rubber component has not less than three functional groups capable of crosslinking reaction in a molecule, it efficiently forms a network structure, whereby the effect of the present invention is sufficiently expressed. From such viewpoint, the number of the functional groups in a molecule of the liquid rubber component is preferably not less than 5, more preferably not less than 8, most preferably not less than 10. On the other hand, when the number of the functional group is too many, since the amount of the necessary crosslinking agent increases, the number of the functional groups in a molecule is preferably not more than 20.

In the present invention, since the liquid rubber component has not less than three functional groups capable of crosslinking reaction in a molecule, a three-dimensional network structure can be formed without the necessity to introduce a branch agent and the like into the main chain of the liquid rubber component, a large amount of an organic liquid component can be contained in the gel composition.

On the other hand, the number of the functional groups in a molecule of the liquid rubber component is preferably not more than 15⁻, more preferably not more than 12. This is because too many numbers of functional groups produces many residual active groups and may influence the components in a gel composition such as a drug and the like, as well as decrease the flexibility of the gel composition, thus resulting in degraded followability to the skin deformation.

The number of the functional groups capable of crosslinking reaction in a molecule of the liquid rubber component is determined as follows. The total number of the functional groups capable of crosslinking reaction in the liquid rubber component in a sample is determined, which is divided by the number average molecular weight of the liquid rubber component to give the number of the functional groups capable of crosslinking reaction per 1 molecule of the liquid rubber component. When the functional group is a carboxyl group, the number of the carboxyl groups in the sample is determined by an acid number measurement method using general potassium hydroxide. The number average molecular weight of the liquid rubber component here means a value obtained under the same conditions as for the above-mentioned weight average molecular weight.

The functional group is not particularly limited and, for example, amino group, carboxyl group, hydroxyl group, chlorosulfone group, ester group, epoxy group isocyanate group, methylol group, sulfonic acid group, mercapto group, and the like can be used. These may be used alone or in a combination of two or more kinds thereof. From the aspects of formation of a crosslink bond with an epoxy compound using the below-mentioned crosslinking agent, carboxyl group and hydroxyl group are preferable.

In the present invention, the gel composition is crosslinked. While the crosslinking treatment is not particularly limited, for example, it is achieved by a physical crosslinking treatment by way of irradiation of UV light, electron beam and the like or a chemical crosslinking treatment using various crosslinking agents.

To prevent an adverse influence on the component in a gel composition, such as a drug, the gel composition is preferably crosslinked by adding a crosslinking agent to the gel composition. Examples of the crosslinking agent include isocyanate compounds such as amine compound, epoxy compound, trifunctional isocyanate and the like, organic peroxide, organic metal salt, metal alcoholate, metal chelate compound, multifunctional compound (multifunctional external-crosslinking agent, monomers for multifunctional internal-crosslinking such as diacrylate, dimethacrylate and the like) and the like, which may be used alone or in a combination of two or more kinds thereof. As the crosslinking agent, epoxy compound is preferable. This is because epoxy group that reacted with the carboxyl group in the liquid rubber component develops hydroxyl group, and the hydroxyl group is reactive with other carboxyl group, which is advantageous to the formation of a network structure.

While the proportion of the crosslinking agent to be blended is not particularly limited as long as a sufficient crosslinking structure can be formed in the gel composition, the ratio of the total number (A) of the functional group of the crosslinking agent in the composition to the total number (B) of the functional group of the liquid rubber in the composition ((A/B)×100)[%] is preferably not less than 50[%], more preferably not less than 80%, most preferably not less than 300%. While the upper limit thereof is not particularly limited, to prevent an adverse influence on the component in the gel composition, it is preferably not more than 500%.

In the present invention, an organic liquid component is combined with the network structure formed by the aforementioned liquid rubber components and enhances flexibility of the gel composition. When a chemical substance such as a drug and the like is contained in the gel composition, the organic liquid component functions to dissolve or disperse the substance therein to afford a gel composition containing the organic liquid. The organic liquid component is not particularly limited as long as it is compatible with the liquid rubber component in the gel composition. Examples thereof include fats and oils such as olive oil, castor oil, lanolin and the like, hydrocarbons such as squalene and liquid paraffin, fatty acid esters such as fatty acid alkyl ester and the like, higher fatty acids such as oleic acid and caprylic acid, pyrrolidone such as N-methylpyrrolidone and N-dodecylpyrrolidone, sulfoxides such as decylmethyl sulfoxide, and the like, which may be used alone or in a combination of two or more kinds thereof.

As in the below-mentioned patch preparation, when the gel composition contains a drug, a fatty acid ester is preferable to promote transdermal absorption of the drug. Examples thereof include fatty acid alkyl diesters such as dioctyl phthalate, diisopropyl adipate, diethyl sebacate and the like, fatty acid alkyl monoesters and the like. To efficiently promote transdermal absorption of the drug, fatty acid alkyl monoester is preferable.

When fatty acid alkyl monoester comprises a fatty acid having an unnecessarily large or small number of carbons, the aforementioned compatibility with synthetic rubber and the like may be degraded or the acid may be volatilized during a heating step for preparation of a preparation. In addition, when they comprise a fatty acid having a double bond in a molecule, oxidative decomposition and the like may be developed to cause problems in the preservation stability.

As the fatty acid alkyl monoester to be used, therefore, a fatty acid alkyl ester composed of saturated or unsaturated higher fatty acid preferably having a carbon number of 12 - 16, more preferably 12 - 14, and saturated or unsaturated lower monadic alcohol preferably having a carbon number of 1 - 4 is preferably employed. Examples of the higher fatty acid alkyl monoester preferably include lauric acid (C12), myristic acid (C14) and palmitic acid (C16), particularly myristic acid and palmitic acid. Examples of the lower monovalent alcohol include methyl alcohol, ethyl alcohol, propyl alcohol and butyl alcohol. They are not limited to straight chain alcohols and may be branched alcohols. Particularly, isopropyl alcohol is preferable. Accordingly, the most preferable fatty acid alkyl esters are isopropyl myristate and isopropyl palmitate.

The proportion of these organic liquid components to be blended is 1,250 parts by weight or below, preferably 1,000 parts by weight or below, more preferably 950 parts by weight or below, more preferably 900 parts by weight or below, more preferably 850 parts by weight or below, more preferably 800 parts by weight or below, more preferably 750 parts by weight or below, more preferably 700 parts by weight or below, most preferably 650 parts by weight or below, relative to 100 parts by weight of the liquid rubber component. When it exceeds 1,250 parts by weight, gel formation may not be available and the composition may not be molded into a given shape.

On the other hand, the proportion of the organic liquid component to be blended is 90 parts by weight or above, preferably 150 parts by weight or above, more preferably 200 parts by weight or above, more preferably 250 parts by weight or above, most preferably 300 parts by weight or above, relative to 100 parts by weight of the liquid rubber component. When it is less than 90 parts by weight, the gel may become too stiff and skin adhesion and skin following capability may be degraded. In addition, diffusion transferability of other components in and absorption thereof by the gel composition may be degraded.

The gel composition of the present invention contains an organic liquid component in an amount equivalent to or greater than that of a liquid rubber component. This may be the reason for the structure wherein the organic liquid component is simply present in the network structure of liquid rubber components, which is formed by crosslinking of the gel composition. Rather, however, it is assumed that the liquid rubber component is present in the organic liquid component in the structure. As a result, the gel composition of the present invention molded into a given shape and applied to the skin as a molded article is considered to show optimal flexibility permitting easy followability and adhesion to the deformation of the skin.

The gel composition may contain other optional components as long as the effect of the present invention is not inhibited. For example, surfactants such as glycerol fatty acid ester, sorbitan fatty acid ester and the like, high boiling point organic solvents such as dimethylsulfoxide, N-methylpyrrolidone and the like, absorption promoters such as pyrrolidonecarboxylate and the like, antioxidants such as ascorbic acid and the like, anti-aging agents, fillers, colorants, softening agents and the like may be contained.

The gel composition of the present invention can be molded into a molded article having a given shape. Examples of the shape of the molded article include sheet preparation, block preparation and the like. For application to medical materials or hygiene materials, a sheet preparation, i.e., a sheet material, is preferable.

Such molded article can be formed into a sheet preparation (sheet material) having a certain thickness of, for example, not less than 0.5 mm, not less than 1 mm, not less than 2 mm or not less than 3 mm and not more than 10 mm. Such sheet material has a greater thickness than that of an adhesive layer of a conventional patch, and is advantageous in that a large amount of an organic liquid component, a chemical substance such as a drug and the like can be contained therein.

For use as a medical material, for example, a patch wherein the molded article of the present invention is formed at least on one surface of a support can be mentioned. Such patch is used for protecting the skin surface and the like. While the support is not particularly limited, one substantially impermeable to the components in the molded article; in other words, a support through which the components in the molded article are not lost from the back face to decrease the content, is preferable. When desired, the adhesive force of the molded article itself can be independently reinforced with an adhesive. When desired, a release liner known per se may be laminated on the surface of the molded article for protection thereof before use.

Moreover, a patch preparation can be obtained by impregnating the molded article in the aforementioned patch with a drug. The drug here is not particularly limited, but a drug that can be administered to a mammal such as human and the like through the skin, i.e., transdermally absorbable drug, is preferable. Examples of the drug specifically include general anesthetics, hypnotic sedatives, antiepileptic drugs, antipyretic analgesic antiphogistic drugs, anti-vertigenous drugs, psychoneurotic drugs, topical anesthetics, skeleton muscle relaxants, autonomic drugs, anti-convulsants, anti-parkinsonian drugs, anti-histamine drugs, cardiac stimulants, drugs for arrhythmia, diuretics, hypotensive drugs, vasoconstrictors, colonary vasodilators, peripheral vasodilators, arteriosclerosis drugs, drugs for circulatory organ, anapnoics, anti-tussive expectorants, hormone drugs, external drugs for mattery diseases, analgesic·antipruritic· styptic·antiphogistic drugs, drugs for parasitic dermatic diseases, drugs for arrest of bleeding, gout treatment drugs, drugs for diabetes, drugs for anti-malignant tumor, antibiotics, chemical therapy drugs, narcotics, quit smoking aids and the like.

The proportion of a drug in a gel composition is not particularly limited as long as it affords the effect of the drug to be transdermally absorbed and does not impair the property of the gel composition. It is preferably 0.1 - 60 wt%, more preferably 0.5 - 40 wt%, of the gel composition. When it is less than 0.1 wt%, the treatment effect may not be sufficient, and when it exceeds 60 wt%, skin irritation may be developed and the proportion may also be economically disadvantageous.

The molded article of the present invention can be specifically used as a matrix of a matrix type patch preparation, a reservoir of a reservoir type patch preparation and the like.

For a hygiene material, a molded article having a given shape, for example, a sheet preparation (sheet material), can be used as a substitute for gauze in an adhesive plaster, a substrate of a gel patch, non-woven fabric substitute for a wound covering dressing and the like.

The gel composition of the present invention can be produced, for example, as follows: a liquid rubber component and an organic liquid component are mixed in, where necessary, the presence of a solvent, a crosslinking agent is added thereto and the mixture is poured into a mold with a given shape. This is heated to a suitable temperature to evaporate the solvent, and the liquid rubber is crosslinked and, where necessary, aged.

### Examples

(Example and Comparative Example)
Carboxylated liquid polyisoprene (weight average molecular weight 25,000) as a liquid rubber component having 10 functional group capable of crosslinking reaction in a molecule and isopropyl myristate as an organic liquid component were mixed at a ratio show in Table 1, a compound (number average molecular weight 366) containing a tetrafunctional epoxy group was blended to meet the "functional group ratio" shown in Table 1. The mixture was stirred and blended to give a composition for forming a gel composition. This was poured into a container having a given shape in a given amount, heated at 120°C for 3 hr for crosslinking, and aged at 80°C for 72 hr to give molded articles of the gel compositions of Examples and Comparative Examples. The "functional group ratio" means the ratio of the total number (A) of the functional group of the crosslinking agent in the composition to the total number (B) of the functional group of the liquid rubber in the composition ((A/B)×100) [%].

**Table 1 Blending rate**

| | liquid rubber component (parts by weight) | organic liquid component (parts by weight) | functional group ratio (%) |
|---|---|---|---|
| Comparative Example 1 | 100 | 80 | 100 |
| Example 1 | 100 | 100 | 100 |
| Example 2 | 100 | 200 | 100 |
| Example 3 | 100 | 400 | 100 |
| Example 4 | 100 | 500 | 100 |
| Example 5 | 100 | 600 | 100 |
| Example 6 | 100 | 700 | 100 |
| Example 7 | 100 | 800 | 100 |
| Example 8 | 100 | 1000 | 100 |
| Comparative Example 2 | 100 | 1500 | 100 |
| Example 9 | 100 | 200 | 200 |
| Example 10 | 100 | 400 | 200 |
| Example 11 | 100 | 500 | 200 |
| Example 12 | 100 | 600 | 200 |
| Example 13 | 100 | 700 | 200 |
| Example 14 | 100 | 800 | 200 |
| Example 15 | 100 | 100 | 400 |
| Example 16 | 100 | 200 | 400 |
| Example 17 | 100 | 400 | 400 |
| Example 18 | 100 | 600 | 400 |
| Example 19 | 100 | 1000 | 400 |
| Example 20 | 100 | 1000 | 800 |
| Example 21 | 100 | 600 | 1000 |
| Example 22 | 100 | 500 | 50 |
| Example 23 | 100 | 600 | 50 |
| Example 24 | 100 | 700 | 50 |
| Example 25 | 100 | 800 | 50 |

(Experimental Example)

### (1) Gel formation

The aforementioned composition for forming a gel composition was poured into a glass bottle (inner diameter 36 mm, depth 60 mm) to a height of about 10 mm, crosslinked as mentioned above, and aged to give molded articles of Examples and Comparative Examples. Whether or not they form gel by crosslinking and whether or not they retain shape were visually evaluated according to the following criteria. Criteria
○ : Gel was formed to maintain a given shape, and the shape was retained even when the glass bottle was tilted 90° or above. Δ: Gel was formed, and the organic liquid component oozed out on the gel surface. ×: Gel was not formed, and the given shape could not be maintained (fluid state). The evaluation results of the gel formation are shown in Table 2.

**Table 2 Gel formation evaluation results**

| | blending rate (parts by weight) of organic liquid component relative to 100 parts by weight of liquid rubber component | | | | | | | | | |
|---|---|---|---|---|---|---|---|---|---|---|
| functional group rate (%) | 80 | 100 | 200 | 400 | 500 | 600 | 700 | 800 | 1000 | 1500 |
| 50 | | | | | Ex. 22 ○ | Ex. 23 ○ | Ex. 24 ○ | Ex. 25 Δ | | |
| 100 | Comp. Ex. 1 ○ | Ex. 1 ○ | Ex. 2 ○ | Ex. 3 ○ | Ex. 4 ○ | Ex. 5 ○ | Ex. 6 ○ | Ex. 7 ○ | Ex. 8 Δ | Comp. Ex. 2 × |
| 200 | | | Ex. 9 ○ | Ex. 10 ○ | Ex. 11 ○ | Ex. 12 ○ | Ex. 13 ○ | Ex. 14 ○ | | |
| 400 | | Ex. 15 ○ | Ex. 16 ○ | Ex. 17 ○ | | Ex. 18 ○ | | | Ex. 19 ○ | |
| 800 | | | | | | | | | Ex. 20 ○ | |
| 1000 | | | | | | Ex. 21 ○ | | | | |

### (2) Flexibility

The aforementioned composition for forming a gel composition was poured into a glass Petri dish (inner diameter 90 mm, depth 18 mm) to a height of 10 mm, and crosslinked and aged as mentioned above. The composition was punched into a circle with a diameter of 30 mm to give a cylindrical sample.

The testing power when a steel ball (diameter 10 mm) was pressed toward the center of the sample for 3 mm at 20 mm/min was measured with a rheo meter. A smaller testing power means that the sample is flexible. A similar measurement was performed with the human skin and the results shown in the following Table 3 "measurement results of testing power with human skin" were obtained. Thus, the following criteria were set, and the flexibility of the gel composition was evaluated.

**Table 3 Measurement results of testing power with human skin**

| part | | testing power (N) |
|---|---|---|
| palm | thick part | 0.26 |
| | thin part | 6.40 |
| backhand | thick part | 0.44 |
| | thin part | 8.58 |
| | thin part | 9.76 |
| forearm | thick part | 0.66 |
| | thin part | 2.46 |
| | thin part | 9.38 |

(Criteria)
⊙ : The testing power is 0.2 or above and less than 8. That is, flexibility is equivalent to that of most parts of human skin, and followability to the skin deformation is extremely high. ○: The testing power is 8 or above and less than 10. That is, flexibility is equivalent to that of somewhat stiff parts of human skin, and followability to the skin deformation is high. Δ: The testing power is 10 or above and less than 14. That is, the sample is somewhat stiff as compared to human skin, and followability to the skin deformation is rather low. However, the sample is similar to the human skin and evaluated to be tolerable. ×: The testing power is 14 or above. That is, the sample is stiff as compared to human skin, and followability to the skin deformation is low.

The evaluation results of flexibility are shown in Table 4.

**Table 4 Flexibility evaluation results**

| | blending rate (parts by weight) of organic liquid component relative to 100 parts by weight of liquid rubber component | | | | | | | | | |
|---|---|---|---|---|---|---|---|---|---|---|
| functional group ratio (%) | 80 | 100 | 200 | 400 | 500 | 600 | 700 | 800 | 1000 | 1500 |
| 50 | | | | | | 0.037 ○ | | | | |
| 100 | 14.21 × | 12.11 Δ | 5.98 ⊙ | 1.50 ⊙ | | 0.302 ⊙ | | | | |
| 200 | | | 7.78 ⊙ | 2.06 ⊙ | | 0.526 ⊙ | | | | |
| 400 | | 9.69 ○ | 6.00 ⊙ | 1.70 ⊙ | | | | | 0.035 ○ | |
| 800 | | | | | | | | | 0.036 ○ | |

As is clear from Table 2, gel was formed in any Example wherein the amount of the organic liquid component was 80 - 1,000 parts by weight relative to 100 parts by weight of the liquid rubber component, and the shape was sufficiently retained. Like Example 25 and Example 8 wherein the amount of the organic liquid component was 800 and 1,000 parts by weight relative to 100 parts by weight of the liquid rubber component, the organic liquid component exuded on the gel surface in some of them depending on the functional group ratio. Comparative Example 2 containing 1,500 parts by weight of the organic liquid component failed to form gel. This means that the blending ratio of the crosslinking agent is preferably increased to raise the functional group ratio depending on the blending ratio of the organic liquid component.

As is clear from Table 4, Examples wherein the amount of the organic liquid component was 100 - 1,000 parts by weight relative to 100 parts by weight of the liquid rubber component showed flexibility similar to that of the human skin, and followability to the skin was high. Particularly, samples wherein the amount of the organic liquid component was 200 - 600 parts by weight showed flexibility equivalent to that of the human skin, and were evaluated to have extremely high followability to the skin.

## Claims

1. A gel composition for a medical material or a hygiene material, which comprises a liquid rubber component comprising, in a molecule, 3 or more functional groups capable of crosslinking reaction, and 90 to 1250 parts by weight of an organic liquid component relative to 100 parts by weight of the rubber component, which gel composition is crosslinked.

2. A composition according to claim 1, comprising the organic liquid component in a proportion of 150 to 1,000 parts by weight relative to 100 parts by weight of the liquid rubber component.

3. A composition according to claim 1 or claim 2, wherein the composition is crosslinked by a crosslinking agent, and the ratio of the total number (A) of the functional groups of the crosslinking agent in the composition to the total number (B) of the functional groups of the liquid rubber in the composition ((A/B)×100) [%] is not less than 50[%].

4. A composition according to any one of claims 1 to 3, wherein the liquid rubber component is a liquid isoprene rubber.

5. A patch comprising a sheet material comprising the composition of any one of claims 1 to 4 on at least one surface of a support.

6. A patch preparation according to claim 5, wherein the sheet material comprises a drug.

## Patentansprüche

1. Gelzusammensetzung für ein medizinisches Material oder ein Hygienematerial, umfassend eine flüssige Kautschukkomponente, die in einem Molekül 3 oder mehr funktionelle Gruppen umfasst, die zu einer Vernetzungsreaktion befähigt sind, und 90 bis 1250 Gewichtsteile einer organischen flüssigen Komponente, bezogen auf 100 Gewichtsteile der Kautschukkomponente, wobei die Gelzusammensetzung vernetzt ist.

2. Zusammensetzung gemäß Anspruch 1, umfassend die organische flüssige Komponente in einem Anteil von 150 bis 1000 Gewichtsteilen, bezogen auf 100 Gewichtsteile der flüssigen Kautschukkomponente.

3. Zusammensetzung gemäß Anspruch 1 oder 2, wobei die Zusammensetzung durch ein Vernetzungsmittel vernetzt ist und das Verhältnis der Gesamtzahl (A) der funktionellen Gruppen des Vernetzungsmittels in der Zusammensetzung zur Gesamtzahl (B) der funktionellen Gruppen des flüssigen Kautschuks in der Zusammensetzung ((A/B) × 100) [%] nicht kleiner als 50% ist.

4. Zusammensetzung gemäß einem der Ansprüche 1 bis 3, wobei die flüssige Kautschukkomponente ein flüssiger Isoprenkautschuk ist.

5. Pflaster, umfassend ein Bahnmaterial, das die Zusammensetzung gemäß einem der Ansprüche 1 bis 4 auf wenigstens einer Fläche eines Trägers umfasst.

6. Pflasterpräparat gemäß Anspruch 5, wobei das Bahnmaterial einen Wirkstoff umfasst.

## Revendications

1. Composition de gel pour un matériau médical ou un matériau hygiénique, qui comprend un composant de caoutchouc liquide comprenant, dans une molécule, 3 groupes fonctionnels ou plus, pouvant subir une réaction de réticulation, et 90 à 1250 parties en poids d'un composant liquide organique par rapport à 100 parties en poids de composant de caoutchouc, ladite composition de gel étant réticulée.

2. Composition selon la revendication 1, comprenant le composant liquide organique en une proportion de 150 à 1000 parties en poids par rapport à 100 parties en poids de composant de caoutchouc liquide.

3. Composition selon la revendication 1 ou la revendication 2, ladite composition étant réticulée par un agent de réticulation et le rapport du nombre total (A) de groupes fonctionnels de l'agent de réticulation dans la composition au nombre total (B) de groupes fonctionnels du caoutchouc liquide dans la composition ((A/B) x 100) [%] n'étant pas inférieur à 50 [%].

4. Composition selon l'une quelconque des revendications 1 à 3, dans laquelle le composant de caoutchouc liquide est un caoutchouc isoprène liquide.

5. Patch comprenant un matériau en feuille comprenant la composition selon l'une quelconque des revendications 1 à 4, sur au moins une surface d'un support.

6. Préparation pour patch selon la revendication 5, dans laquelle le matériau en feuille comprend un médicament.
